# EUROPEAN PATENT APPLICATION

(11) **EP 4 576 106 A1**
(43) Date of publication of application: **25.06.2025**
(21) Application number: 24155921.0
(22) Date of filing: 06.02.2024
(51) Int. Cl.: G16H 10/60, G16H 50/20, G16H 50/30, G16H 10/20

(54) **SYSTEMS, METHODS, AND ARTICLES FOR STRUCTURED ELECTRONIC HEALTH RECORD IMPUTATION USING DIAGNOSTIC TEMPORAL WINDOW**

(30) Priority: 18.12.2023 US 202318544092
(71) Applicant: Tempus AI, Inc., Chicago, IL 60654 (US)
(72) Inventor: Fernandes, Louis, Park Ridge, 60068 (US); Ferguson, Javid, Chicago, 60625 (US)
(74) Representative: Eisenführ Speiser

(57) **Abstract**

Systems, methods, and apparatuses for imputing a value associated with a subject within an electronic health record (EHR) system are disclosed herein. A request to impute a value associated with the subject at a diagnostic temporal instance is received, and a subset of data associated with the subject from an EHR system is retrieved. One or more temporal windows are determined and used to select health observations having a temporal instance within the one or more temporal windows. Values corresponding to the selected health observations are retrieved as input values, which are provided as input to a trained artificial intelligence engine. The trained artificial intelligence engine processes the input values to generate the imputed value and provides the imputed value in response to the request.

## Description

### BACKGROUND

### Technical Field

The present disclosure generally relates to imputing missing values in an Electronic Health Record (EHR), and more particularly, to using one or more temporal diagnostic windows and artificial intelligence to impute values.

### Description of the Related Art

An electronic health record (EHR) contains information relating to patient care, often from a variety of sources. In many cases, an EHR contains unstructured data. Unstructured data does not have a predefined data model, and may include information in many different formats, many of which cannot be processed or analyzed using conventional methods.

Medical notes are one example of unstructured data. Medical notes document a patient's interaction with a healthcare worker such as a physician, nurse, physician's assistant, technician, radiologist, or the like, and may be stored in an EHR. Medical notes may include consultation notes, referral notes, Subject, Objective, Assessment, and Plan (SOAP) notes, procedure notes, phone notes, or the like. Medical notes are often handwritten and may use non-standard formatting. Data such as x-ray images, mammograms, and digital pathology files often appear as unstructured data in an EHR.

While unstructured data may comprise detailed information about a patient encounter, factors including inconsistent formatting, quality, and data types may make unstructured data difficult to interpret and extract information from systematically.

Electronic health records may also include structured data. In contrast to unstructured data, structured data is organized into fields in a database or other organizational schema. Examples of structured data include demographic information about a subject such as the subject's address, age, weight, and height. Diagnostic codes, billing codes, and laboratory test results often appear as structured data in electronic health records.

A clinical trial may be used to test the efficacy of a therapy in a clinical setting. To be included in a clinical trial, a patient must meet eligibility criteria. Eligibility criteria may include inclusion criteria, exclusion criteria, or a combination thereof. The terms "inclusion criteria" and "exclusion criteria" may at times be used interchangeably, wherein an exclusion criterion may be stated in terms of an inclusion criterion. For example, a clinical trial may state that an exclusion criterion is a subject having an age less than eighteen. This may be stated as an inclusion criterion, wherein the inclusion criterion is met if the patient has an age of 18 years or older. Therefore, the term "inclusion criteria" as used herein may include exclusion criteria stated as inclusion criteria and vice versa, and the term "inclusion criterion" may include an exclusion criterion stated as an inclusion criterion and vice versa.

For example, matching a subject (patient) with a clinical trial may be difficult using unstructured data or structured data in an EHR system and traditional methods. Many clinical trials for cancer therapies, for example, have an eligibility requirement specifying a particular stage of cancer. Cancer stage is rarely present in structured electronic health record data. Therefore, subjects who might in fact be eligible for the clinical trial for cancer therapy cannot be matched with the trial because one or more eligibility criteria cannot be confirmed in EHR system data. In some cases, a subject who may be eligible but for missing EHR system data may not even be aware they are likely to be eligible, or that their eligibility could be confirmed with a diagnostic test.

### BRIEF SUMMARY

Systems, methods, and articles for imputing a value associated with a subject within an EHR system are provided. In some cases, a clinical trial is selected, wherein the subject meets one or more eligibility criteria of the clinical trial. In these cases, a missing value in the data associated with the subject from the EHR system is determined. The missing value relates to an eligibility criterion for the clinical trial not met by the subject. The value to impute may have a relevance to making a risk determination.

A request to impute a value associated with the subject at a diagnostic temporal instance is received. According to some embodiments where the clinical trial is selected, the missing value is the value to impute. Then, a subset of data associated with the subject from the EHR system is retrieved. The subset of data is organized into specific fields as part of a schema and includes a plurality of health observations. Each health observation is associated with a temporal instance. In some cases, a second subset of data is retrieved, wherein each second health observation in the second subset is not associated with a temporal instance. The health observations may be selected based on a relevance to an extent of tumor, extent of spread to lymph nodes, and presence of metastasis (TNM) staging system. The health observations may also be selected based on a variance of a value corresponding to a feature in the health observation across health observations in the EHR system having the feature. In some cases, health observations having a feature with a variance across the EHR system above a predetermined threshold are selected.

For each health observation associated with a temporal instance, a pre-diagnostic temporal window and a post-diagnostic temporal window are determined according to some embodiments. The pre-diagnostic temporal window comprises a specified first duration immediately preceding the diagnostic temporal instance, while the post-diagnostic temporal window comprises a specified second duration immediately following the diagnostic temporal instance. The pre-diagnostic temporal window and/or the post-diagnostic temporal window may be based on a characteristic or survival rate of a condition associated with the value to impute. The pre-diagnostic temporal window and/or the post-diagnostic temporal window may also be based on a diagnostic window hyperparameter. Then, health observations having a temporal instance within the pre-diagnostic temporal window or the post-diagnostic temporal window are selected.

For each of the selected health observations, a value corresponding to the selected health observation is retrieved. These retrieved values are input values. Values corresponding to each second health observation may also be retrieved and added to the input values. A subset of the input values may be associated with the same feature. In some cases, the subset is determined, and a representative value is calculated based on the subset. Then, the subset is removed from the input values and the representative value is added to the input values.

A temporal sub-window may be calculated. The temporal sub-window may comprise a portion of the pre-diagnostic temporal window, a portion of the post-diagnostic temporal window, or both. A bucket of values is determined, the bucket of values including each input value in the subset corresponding to a health observation having a temporal instance in the temporal sub-window. Then, a count of the values in the bucket of values is determined. The bucket of values is removed from the input values and the count is added to the input values.

In some cases, the above steps are stored on non-transitory computer-readable media and performed with a processor of a computing system. In some cases, the above steps are stored in a non-transitory processor-readable storage medium.

The input values are provided to a trained artificial intelligence engine. The trained artificial intelligence engine processes the input values to generate the imputed value and provides the imputed value in response to the request.

According to some embodiments where a clinical trial is selected, the imputed value is used to automatically perform a preliminary matching of the subject with the clinical trial based on the imputed value. In some cases, the imputed value is related to an eligibility criterion of the clinical trial. Then, an appropriate diagnostic test to confirm the preliminary matching is determined. A message is sent to the subject, wherein the message is based on the preliminary matching and the appropriate diagnostic test to confirm the preliminary matching.

In some cases, a cancer stage is determined based on the imputed value. The cancer stage may conform with a TNM staging system, which assesses an extent of a tumor, a degree of spread to lymph nodes, and a presence of metastasis. In some cases, the imputed value is the cancer stage. A risk determination may be based on the imputed value.

A request to impute a second value associated with the subject at a second diagnostic temporal instance may be received. Then, the imputed value is added to the input values, which are provided again to the trained artificial intelligence engine, which processes the input values to generate the second imputed value and provides the second imputed value.

Embodiments of the present disclosure improve the functioning of computers by solving the technical problem of data missingness in structured EHR data. As discussed above, structured EHR data may only contain basic information related to billing, lab test results, or the like. Because structured EHR data is structured, it is less computationally expensive to search and perform analysis on than unstructured EHR data. Embodiments of the present disclosure provide for imputing values useful for making clinical decisions from structured EHR data. This alleviates the need to search for information such as a cancer stage value in unstructured EHR data, which may require heterogenous and expensive data processing techniques to extract useful information from. Therefore, the computational resources may be deployed for other tasks, improving the functioning of computers.

Additionally, the use of diagnostic temporal windows according to embodiments of the present disclosure increases the relevance of structured EHR data used to train the artificial intelligence engine. By removing health observations having a temporal instance outside one or more temporal windows based on a characteristic of a condition associated with the value to impute, the trained artificial intelligence engine is not trained using health observations with low correlation to the value to impute. Accordingly, the computing resources necessary to train the trained artificial intelligence engine are reduced, freeing the computing resources to be deployed for other tasks. The performance of computers implementing embodiments of the present disclosure is thereby improved.

### BRIEF DESCRIPTION OF THE SEVERAL VIEWS OF THE DRAWINGS

Like-numbered elements may refer to common components in the different figures.
Figure 1 depicts a system for imputing a value associated with a subject within an electronic health record (EHR) system according to some embodiments of the present disclosure.
Figure 2A depicts a pre-diagnostic temporal window and a post-diagnostic temporal window according to some embodiments of the present disclosure.
Figure 2B depicts a diagnostic temporal window according to some embodiments of the present disclosure.
Figure 2C depicts a diagnostic temporal window according to some embodiments of the present disclosure.
Figure 2D depicts a pre-diagnostic temporal window and a post-diagnostic temporal window with sub-windows according to some embodiments of the present disclosure.
Figure 3A depicts an example feature set produced using various temporal windows according to some embodiments of the present disclosure.
Figure 3B depicts an example subject health observation set according to some embodiments of the present disclosure.
Figure 3C depicts an example subject feature vector according to some embodiments of the present disclosure.
Figure 4 depicts a flowchart describing a process for imputing a value associated with a subject within an electronic health record (EHR) system according to some embodiments of the present disclosure.
Figure 5 depicts a flowchart describing performing a preliminary clinical trial matching using an imputed value according to some embodiments of the present disclosure.
Figure 6 depicts a flowchart describing a process for aggregating a subset of input values into a representative value according to some embodiments of the present disclosure.
Figure 7 depicts selected elements of a system for imputing a value associated with a subject within an electronic health record (EHR) system according to some embodiments of the present disclosure.

### DETAILED DESCRIPTION

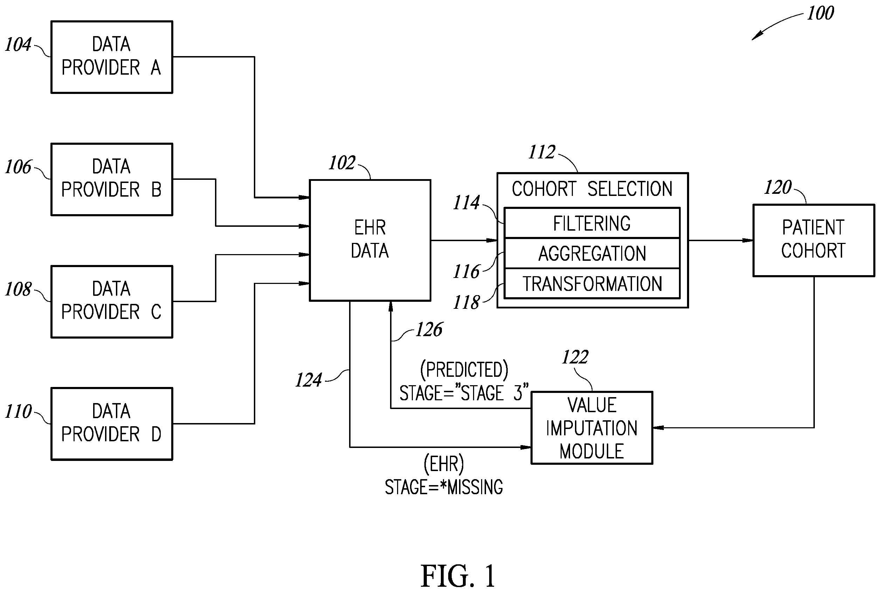

Figure 1 depicts a system 100 for imputing a value associated with a subject within an electronic health record (EHR) system according to some embodiments of the present disclosure. Data provider A 104, data provider B 106, data provider C 108, and data provider D 110 (data providers) supply data to EHR data 102. A data provider may be a hospital, healthcare provider, pharmacy, or the like. As discussed above, EHR data may be structured or unstructured and may include medical notes, scans, billing codes, diagnostic codes, laboratory test results, demographic information, or the like. EHR data 102 is the aggregated EHR data from the data providers.

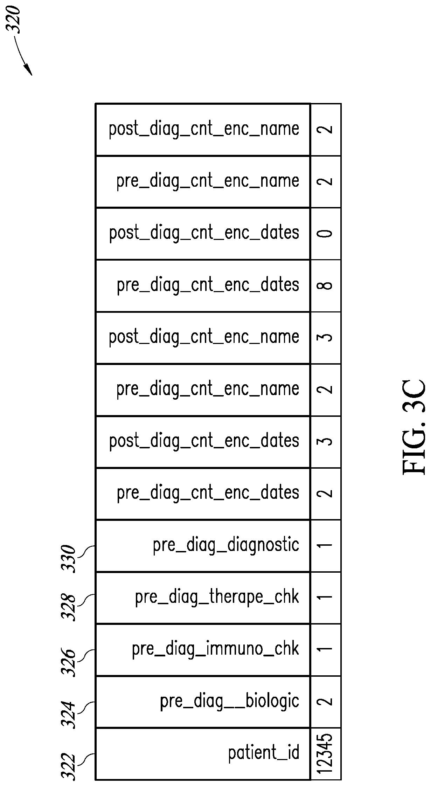

To impute a value associated with a subject within an EHR system, EHR data 102 is processed into patient cohort 120. Patient cohort 120 includes a plurality of patient feature vectors corresponding to a plurality of patients in EHR data 102. Each patient feature vector includes one or more features, and each patient feature vector in a particular patient cohort 120 may include the same features. Patient feature vectors are described in detail with respect to Figure 3C. To create patient cohort 120, EHR data 102 is processed into patient cohort 120 at cohort selection 112 which may include one or more of filtering 114, aggregation 116, or transformation 118.

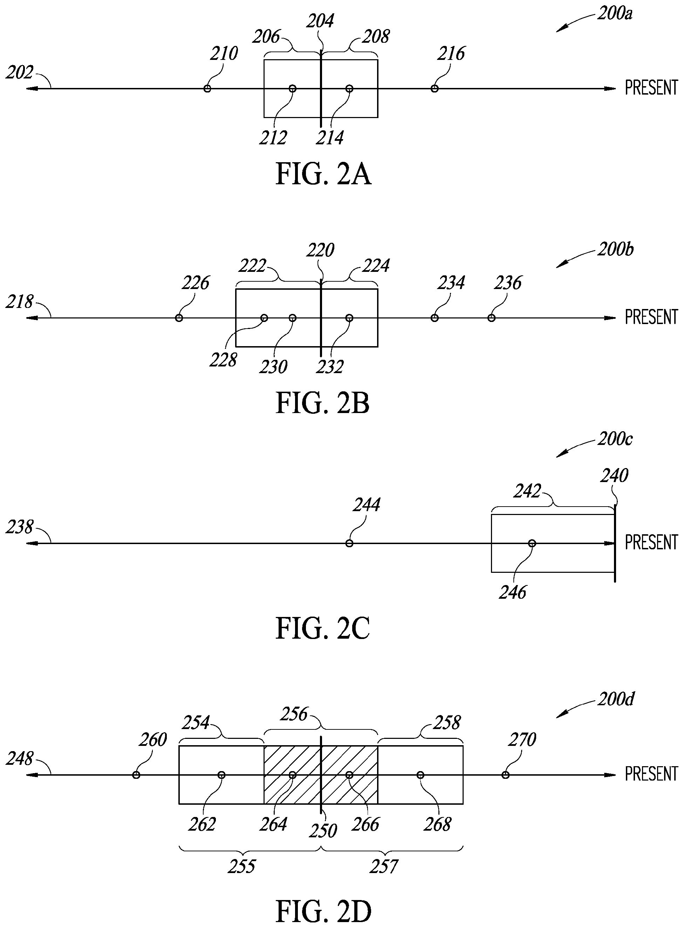

Filtering 114 includes calculating and implementing one or more temporal windows. For example, a pre-diagnostic temporal window and the post-diagnostic temporal window may be determined relative to a diagnostic temporal instance received with the request to impute the value. The pre-diagnostic temporal window comprises a specified duration immediately preceding the diagnostic temporal instance, while the post-diagnostic temporal window comprises a specified second duration immediately following the diagnostic temporal instance. Calculating and implementing the one or more temporal windows is discussed in detail with respect to Figures 2A-2D.

Filtering 114 according to some embodiments comprises removing erroneous observations from the EHR data. Health observations with missing values, incorrect values, and other defects may be removed from the EHR data. Filtering 114 may also include selecting each patient having a feature in EHR data 102 corresponding to the feature of the value to impute. For example, if the value to impute is a cancer stage according to the TNM staging system, each patient having a feature corresponding to a cancer stage according to the TNM staging system may be selected.

According to some embodiments, filtering 114 applies one or more filtering methods including Pearson's correlation, linear discriminant analysis, analysis of variance, chi-square, or similar methods to select features having a desired degree of correlation with the value to impute from EHR data 102. According to some embodiments, filtering is additionally or alternatively performed by machine learning methods implemented by value imputation module 122, for example, when lasso regression or ridge regression are used.

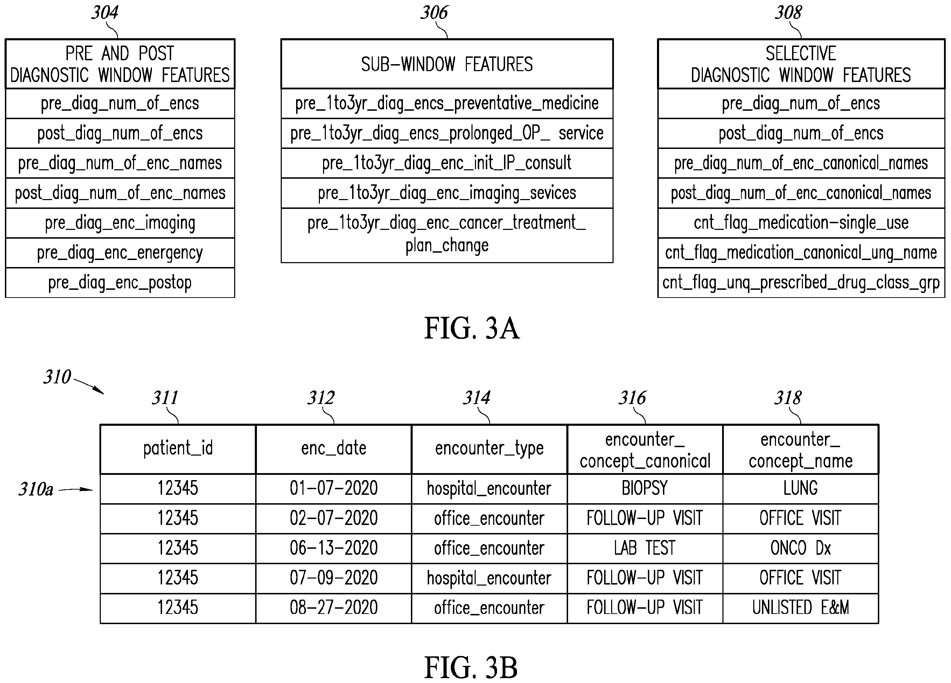

Aggregation 116 may include compiling observations from EHR data 102 into patient feature vectors as described with respect to Figures 3A-3C. For example, a plurality of health observations corresponding to a patient may be arranged into a patient feature vector for use by value imputation module 122. Aggregation 116, according to some embodiments, includes calculating a representative value based on a subset of EHR data 102 corresponding to a patient. The patient may have health observations including multiple values for the same feature at different times. For example, a patient may have multiple features corresponding to a cancer stage, resulting from a plurality of healthcare encounters in a temporal period. Depending upon a temporal instance the cancer stage is assessed, it may be stage 0, stage 1, stage 2, stage 3, or stage 4. Therefore, the EHR data corresponding to the patient may include several different cancer stage values. But according to some embodiments, only one cancer stage value is required. According to some embodiments, the representative value is the most recent cancer stage value or is calculated by taking the most severe cancer stage from the EHR data corresponding to the patient. According to some embodiments, the representative value is a largest, smallest, mean, mode, median, or weighted average of the values corresponding to a feature in the EHR data corresponding to the patient. A representative value may be calculated for any set of health observations corresponding to the same feature.

Additionally, the representative value may be computed using a plurality of values corresponding to a feature from EHR data 102. A value may be replaced with a percentile or quantile of the value as compared to one or more values corresponding to the same feature in the dataset. For example, a value corresponding to a feature of white blood cell count per microliter of blood may be 2,000. If 2,000 white blood cells per microliter of blood corresponds to a 5^{th} percentile as compared to other values corresponding to that feature, the value 2,000 may be replaced with 5. The plurality of values corresponding to the feature from EHR data 102 may include every value in the set of values corresponding to the feature in EHR data 102, or any subset thereof. The representative value may be calculated based on any suitable data summary method as applied to the plurality of values corresponding to the feature from EHR data 102, including temporal associations, frequency table, tail proportion, quantile, order statistic, interquartile range, interdecile range, median absolute deviation, quantile skewness, skew, variance, standard deviation, or any other moment or quantile-based summary statistic.

Transformation 118 may include transforming observations from EHR data 102 for data type compatibility. EHR data 102 may include nonnumeric features such as strings, which may be incompatible with value imputation module 122, which may require numeric values. For example, a value corresponding to a feature of cancer stage may appear as the string "stage 3" in EHR data 102. Therefore, to use the value with value imputation module 122, the string "stage 3" may be converted into the integer 3 or encoded into a standardized representation for the structured EHR.

According to some embodiments, patient cohort 120 comprises a plurality of patient feature vectors. According to some embodiments, patient cohort 120 may include a set of training examples. In one example, each training example of the set of training examples may include an input-output pair, such as a pair comprising an input vector and a target answer. The input vector may be a patient feature vector, and the target answer may be the value of a feature associated with the patient.

Value imputation module 122 may implement a supervised machine learning algorithm using patient cohort 120 as training data. Supervised machine learning refers to machine learning methods where labeled training data is used to train or generate a machine learning model or set of mapping functions that maps input feature vectors to output predicted answers. The trained machine learning model may then be deployed to map new input feature vectors to predicted answers. Supervised machine learning may be used to solve regression and classification problems. Regression relates a dependent variable, such as the value to impute, to one or more independent variables, such as the values in a subject feature vector. Regression algorithms include polynomial regression and logistic regression. A classification problem is where the output predicted answer comprises a label, or an identification of a particular class. Classification algorithms may include support vector machine, decision tree, k-nearest neighbor, and random forest algorithms. In some cases, a support vector machine algorithm may determine a hyperplane, or decision boundary, that maximizes the distance between data points for two different classes. The hyperplane may separate the data points for the two different classes and a margin between the hyperplane and a set of nearest data points, or support vectors, may be determined to maximize the distance between the data points for the two different classes.

During a training phase, a machine learning model may be trained to generate predicted answers using a set of labeled training data. According to some embodiments, the set of labeled training data is curated training data. Curated training data may be created by methods including removing irrelevant or redundant data, correcting errors, filling in missing data, formatting the data, or any combination thereof. For example, in the case of cancer stage imputation, a cancer stage corresponding to one or more feature vectors may be extracted from clinical notes.

Training data may be stored in a memory. According to some embodiments, labeled data such as patient cohort 120 may be split into a training data set and an evaluation data set prior to or during the training phase.

Value imputation module 122 may implement a machine learning algorithm that uses patient cohort 120 to train the machine learning model to predict a feature associated with patient cohort 120, such as cancer stage, and uses the evaluation data set to evaluate the predictive ability of the trained machine learning model. The predictive performance of the trained machine learning model may be determined by comparing predicted answers generated by the trained machine learning model with the target answers in the evaluation data set or ground truth values. According to some embodiments, the machine learning algorithm may include a loss function and an optimization technique. The loss function may quantify the penalty that is incurred when a predicted answer generated by the machine learning model does not equal the appropriate target answer. The optimization technique may seek to minimize the quantified loss. One example of an appropriate optimization technique is online stochastic gradient descent.

Value imputation module 122 includes an artificial intelligence engine that categorizes input features in patient cohort 120 into a plurality of classes. The one or more machine learning models included in value imputation module 122 may be utilized to perform binary classification, where an input feature vector is assigned to one of two classes. According to some embodiments, multi-class classification, where an input feature vector is assigned to one of three or more classes is used. The output of the binary classification may comprise a prediction score that indicates the probability that an input feature vector belongs to a particular class. According to some embodiments, a binary classifier may be a function used to decide whether an input feature vector, like a vector of numbers representing the input features, should be assigned to a first class or a second class. The binary classifier may use a classification algorithm that outputs predictions based on a linear predictor function combining a set of weights with the input feature vector. For example, the classification algorithm may compute the scalar product between the input feature vector and a vector of weights and then assign the input feature vector to the first class if the scalar product exceeds a threshold value.

The number of input features, or input values, of a labeled data set may be referred to as its dimensionality. According to some embodiments, dimensionality reduction may be used to reduce the number of input features that are used for training a machine learning model. The dimensionality reduction may be performed using one or more of filtering module 114, aggregation module 116, or transformation module 118. Dimensionality reduction reduces the dimensionality of the feature space by selecting a subset of the most relevant features from an original set of input features.

Feature extraction may also be used. Feature extraction reduces the dimensionality of a feature space by deriving a new feature subspace from the original set of input features. With feature extraction, new features may be different from the input features of the original set of input features and may retain most of the relevant information from a combination of the original set of input features. In one example, feature selection may be performed using sequential backward selection and unsupervised feature extraction may be performed using principal component analysis.

The artificial intelligence engine may be trained using one or more training or learning algorithms. For example, backpropagation may be used to train a multi-layer neural network. According to some embodiments, supervised training techniques using a set of labeled training data may be performed. According to some embodiments, the artificial intelligence engine is trained with unsupervised training techniques using a set of unlabeled training data. One or more generalization techniques may be used to improve the generalization capability of the machine learning models being trained, such as weight-decay and dropout regularization.

Value imputation module 122 receives input 124 specifying a value to impute at a diagnostic time. Using the methods described above, value imputation module 122 processes patient cohort 120 to produce imputed value 126. Imputed value 126 is according to some embodiments added to the EHR data, where it may be used to impute other values.

Figures 2A, 2B, 2C, and 2D depict pre-diagnostic temporal windows and post-diagnostic temporal windows according to various embodiments of the present disclosure. As discussed herein, one or more temporal windows may be used to add temporal sensitivity to value imputation. In some cases, health observations with temporal instances relatively distant from the diagnostic temporal instance are available, such as health observations from 20 years ago. But if the value to be imputed is whether a person currently has influenza, for example, it may be unhelpful or even detrimental to consider health observations from 20 years ago. These health observations may have little or no correlation to whether the person currently has influenza. In some cases, such as imputing a cancer stage, the temporal range of health observations that may be correlated may be much larger. For example, health observations including family history, genetic testing results, etc., for a patient may be relevant regardless of their temporal instances, while health observations including medications, treatments, procedures, tumor information, etc., may be relevant if the health observation has a temporal instance within a few years of the current cancer stage imputation. Accordingly, the temporal window used may be based on a characteristic of a condition associated with the value to impute. The characteristic may be whether the disease has an acute, subacute, or chronic onset, a progression timeline of the condition, or the like. The temporal window may in some embodiments be based on an availability of data in a temporal window.

Figure 2A depicts timeline 200a wherein the leftmost point of line 202 is the point on timeline 200a furthest in the past and the rightmost point on line 202 is the moment at which the windows are determined. Diagnostic temporal instance 204 is an instance in time from which the windows are calculated. According to some embodiments, pre-diagnostic temporal window 206 comprises a specified first duration immediately preceding diagnostic temporal instance 204. Post-diagnostic temporal window 208 comprises a specified second duration immediately following diagnostic temporal instance 204. Health observations 210, 212, 214, and 216 are health observations of the patient with temporal instances corresponding to their respective positions on line 202. In embodiments according to Figure 2A, health observations 210 and 216 are outside the windows 206 and 208, while health observation 212 is within the pre-diagnostic temporal window 206 and health observation 214 is within the post-diagnostic temporal window 208. In this case, health observations 212 and 214 would be selected for use in the input data, while health observations 210 and 216 would not be selected for use.

According to some embodiments, the first duration, the second duration, or both, are based on a diagnostic window hyperparameter. The diagnostic window hyperparameter is according to some embodiments a scalar corresponding to a duration of time. Any suitable unit of time may be used. Thus, the diagnostic window hyperparameter could be 5, as in 5 years, 72, as in 72 months, 31,536,000, as in 31,536,000 seconds, and so on.

Figure 2B depicts another timeline 200b, corresponding to some embodiments of diagnostic temporal windows. The leftmost point of line 218 is the point on timeline 200a furthest in the past and the rightmost point on line 218 is the moment at which the diagnostic temporal windows are determined. Diagnostic temporal instance 220 is an instance in time from which the windows are calculated. According to some embodiments, pre-diagnostic temporal window 222 comprises a specified first duration immediately preceding diagnostic temporal instance 220. Post-diagnostic temporal window 224 comprises a specified second duration immediately following diagnostic temporal instance 220. Health observations 226, 228, 230, and 232, 234, and 236 are health observations of the patient with temporal instances corresponding to their respective positions on line 218. In some embodiments according to Figure 2B, health observations 226, 234, and 236 are outside the windows, while health observations 228 and 230 are within the pre-diagnostic temporal window 222 and health observation 232 is within the post-diagnostic temporal window 224. In this embodiment, health observations 228, 230, and 232 would be selected for use in the input data, while health observations 226, 234, and 236 would not. In some embodiments according to Figure 2B, the pre-diagnostic temporal period is longer than the post-diagnostic temporal period.

Figure 2C depicts timeline 200c, corresponding to some embodiments of a diagnostic temporal window. The leftmost point of the line 238 is the point on timeline 200c furthest in the past and the rightmost point on line 238 is the moment at which the windows are determined. Diagnostic temporal instance 240 is an instance in time from which the windows are calculated. According to some embodiments, pre-diagnostic temporal window 242 comprises a specified first duration immediately preceding diagnostic temporal instance 240. In embodiments according to Figure 2C, there is no post-diagnostic window or, equivalently, the post-diagnostic window has a duration of zero, because the diagnostic temporal instance 240 is at the rightmost point in the timeline. Health observations 244 and 246 are health observations of the patient with temporal instances corresponding to their respective positions on line 238. In embodiments according to Figure 2C, health observation 244 is outside the pre-diagnostic temporal window 242, while health observation 246 is in the pre-diagnostic temporal window. In this case, health observation 246 would be selected for use in the input data, while health observation 244 would not. While Figure 2C depicts a pre-diagnostic time window only, in some embodiments only a post-diagnostic time window is used. Stated differently, the post-diagnostic time window in this example has a duration of zero.

Figure 2D depicts timeline 200d, corresponding to some embodiments of a pre-diagnostic temporal window and a post-diagnostic temporal window with sub-windows. According to some embodiments shown in Figure 2D, each sub-window corresponds to a different feature that may appear in a patient feature vector. For example, one feature may relate to diagnostic scans in the period 1 to 3 years immediately preceding the diagnostic temporal instance, while another feature may relate to diagnostic scans in the period 1 to 3 years immediately following the diagnostic temporal instance. Use of sub-windows is discussed in detail with respect to sub-window features 306 in Figure 3A.

The leftmost point of line 248 is the point on the timeline furthest in the past and the rightmost point on line 248 is the moment at which the windows are determined. Diagnostic temporal instance 250 is an instance in time from which the windows are calculated. First sub-window 254 is within pre-diagnostic temporal window 255. Second sub-window 256 is within both pre-diagnostic temporal window 255 and post-diagnostic temporal window 257. Third sub-window 258 is within post-diagnostic temporal window 257. Health observations 260, 262, 264, 266, 268, and 270 are health observations of the patient with temporal instances corresponding to their respective positions on line 248. In embodiments according to Figure 2D, health observations 260 and 270 are outside the sub-windows, while health observation 262 is within first sub-window 254, health observations 264 and 266 are within second sub-window 256, and health observation 268 is within third sub-window 258. In this case, a first feature would be based on health observation 262, a second feature would be based on health observations 264 and 266, and a third feature would be based on health observation 268.

Figure 3A depicts a feature set produced using various temporal windows according to some embodiments of the present disclosure. As described with respect to Figures 2A-2D, various temporal windows may be used to select health observations to base one or more features on. Pre- and post-diagnostic window features 304 are an example of features resulting from the use of a pre-diagnostic temporal window and a post-diagnostic temporal window, as described with respect to Figures 2A-2B. According to some embodiments, pre_diag_num_of_encs is a feature corresponding to a number of encounters in the pre-diagnostic temporal window, while post_diag_num_of_encs is a feature corresponding to a number of encounters in the post-diagnostic temporal window. Similarly, pre_diag_num_of_enc_names is a feature corresponding to a number of encounter names in the pre-diagnostic temporal window, while post_diag_num_of_enc_names is a feature corresponding to a number of encounter names in the post-diagnostic temporal window.

Sub-window features 306 depict an example feature set produced according to some embodiments having sub-windows. Each feature corresponds to one of one or more sub-windows, as described with respect to Figure 2D. For example, pre_lto3yr diag encs_preventative medicine corresponds to the number of preventative medicine encounters in the sub-window from 1 to 3 years before the diagnostic temporal instance.

Selective diagnostic window features 308 depict an example feature set produced using an embodiment wherein one or more features are not subject to filtering by temporal windows, and one or more other features are subject to filtering by temporal windows. For example, the feature pre_diag_num_of_encs is a number of encounters in the pre-diagnostic temporal window. But cnt_flag_medication_single_use is a number of medications across all available health observations of the subject. Any portion of features may be subject to filtering by temporal window according to various embodiments.

Figure 3B depicts an example subject health observation set 310. Row 310a corresponds to a health observation for a patient having patient_id 311 of 12345. Each health observation depicted in Figure 3B also includes features enc_date 312, encounter_type 314, encounter_concept_canonical 316, and encounter_concept_name 318. Given a diagnostic temporal instance of Jan 1, 2023, and a 3-year pre-diagnostic temporal window, subject health observation set 310 may be processed into a feature corresponding to the number of encounters in the pre-diagnostic temporal window. Using the stated diagnostic temporal instance and pre-diagnostic temporal window, the feature would be five, because there are five encounters in the pre-diagnostic temporal window. Similarly, the value of a feature only considering hospital encounters in the pre-diagnostic temporal window would be two, because there are two hospital encounters in the pre-diagnostic temporal window.

Figure 3C depicts an example subject feature vector for a patient having patient_id 322 of 12345. Subject feature vector 320 includes features 324, 326, 328, and 330. Each of the features are within a pre-diagnostic temporal window and may be calculated similarly to the example discussed with respect to Figure 3B above.

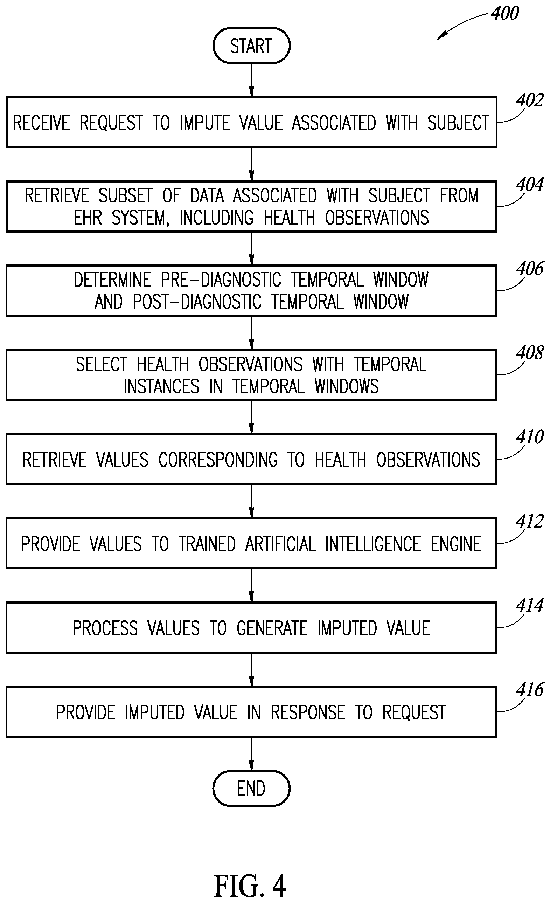

Figure 4 depicts a flowchart describing a process for imputing a value associated with a subject within an electronic health record (EHR) system according to some embodiments of the present disclosure. According to some embodiments, process 400 may be performed using one or more real or virtual machines and/or one or more containerized applications.

Process 400 begins at step 402, where a request to impute a value associated with the subject is received. The request may be provided manually by user input, or automatically. The value to impute may be a cancer stage, a genetic alteration, a genetic variant, a histology, a disease attribute, a potential biomarker status, or the like. The request, according to some embodiment, comprises a feature vector for the subject similar to patient feature vector 320 in Figure 3C.

According to some embodiments, the value to impute may be a value corresponding to an existing data field in the EHR system. For example, the value to impute may be the subject's age where a field corresponding to the subject's age is present but missing data. According to some embodiments, the value to impute may be a value that is present for the subject in the EHR system. This may occur when a value associated with the subject is outside of a range of typical values of the same feature in the EHR system. For example, the Eastern Cooperative Oncology Group (ECOG) performance status scale ranges from zero to five. A patient having a score of zero on the scale is fully active and able to carry out all pre-disease functions, while a patient having a score of five on the scale is dead. If an ECOG score for a patient is recorded as ten, the value to impute may be the ECOG score because the recorded score falls outside the range of the scale and is erroneous. In some embodiments, the value associated with the subject is compared to corresponding values for other subjects in the EHR system. The value associated with the subject is then taken as the value to impute when the value associated with the subject is greater than a threshold, for example, three standard deviations from the mean of the corresponding values. In various embodiments, any suitable statistical measure comparing the value associated with the subject and the corresponding values for other subjects may be used, and any suitable threshold value may be used. In some embodiments, the value to impute does not correspond to any existing field for the subject in the EHR system. For example, the value to impute may be the presence of a biomarker for which a corresponding field does not exist for the subject in the EHR system. In general, any value relating to one or more values in the EHR system may be the value to impute.

Process 400 continues from step 402 to step 404, where a subset of data associated with the subject, including health observations, is retrieved from the EHR system.

Following step 404, in step 406, a pre-diagnostic temporal window and a post-diagnostic temporal window are determined. Various embodiments of the temporal windows are discussed in detail above with respect to Figures 2A-2D. The temporal windows determine which health observations are used, examples of which can be seen in Figures 3A-3B.

Continuing from step 406, in step 408 health observations having temporal instances in the temporal windows are selected. According to some embodiments, the temporal instance of a health observation is a date such as the dates in column 312 of Figure 3B.

Process 400 continues from step 408 to step 410, where values corresponding to each selected health observation are retrieved as input values. As described with respect to Figures 3A-3C, a health observation according to one embodiment includes a patient ID, an encounter date, canonical encounter concept, and an encounter concept name. If the encounter date of the health observation falls within a selected temporal window, the value corresponding to the health observation is used.

After step 410, in step 412, the input values are provided to a trained artificial intelligence engine. According to some embodiments, the trained artificial intelligence engine is a multi-output classifier. The trained artificial intelligence engine may use any known artificial intelligence model, including logistic regression or other types of one or more models. In embodiments where the artificial intelligence model includes logistic regression, the variant of logistic regression used may include multi-label, ordinal, ridge, lasso, or any other known variant of logistic regression. According to some embodiments, the trained artificial intelligence engine may include a random forest classifier. The trained artificial intelligence engine may, according to some embodiments, include an artificial neural network, or any other backpropagation-based machine learning model.

The trained artificial intelligence engine is trained using data such as patient cohort 120 in Figure 1. According to some embodiments, the trained artificial intelligence engine is trained using supervised learning. In these embodiments, labels may be generated for the training data using unstructured EHR data.

After step 412, process 400 continues to step 414, wherein the trained artificial intelligence engine processes the input values to generate the imputed value. As discussed above with respect to Figure 1, the trained artificial intelligence engine may implement any known artificial intelligence algorithm, including polynomial regression, logistic regression, support vector machine, decision tree, k-nearest neighbor, random forest, and neural network. Generating the imputed value according to some embodiments comprises predicting a plurality of values. For example, according to some embodiments of cancer stage imputation, the trained artificial intelligence engine predicts a likelihood of each stage of cancer according to a cancer staging system. Accordingly, a variable corresponding to a likelihood of each of stage 0, stage 1, stage 2, stage 3, and stage 4 given an input subject feature vector, like subject feature vector 320 in Figure 3C, is generated according to some cancer staging systems. The maximum of these values is taken to be the imputed value in some cases.

As discussed above, any number of variables may be predicted in generating the imputed value. According to some embodiments, a variable may represent one or more cancer stages. For example, one predicted value may correspond to a likelihood of a presence of any of cancer stages 0, 1, and 2, while the other predicted value corresponds to a likelihood of a presence of cancer stages 3 and 4. Any combination of groupings may be used according to various embodiments, such as a first variable corresponding to a likelihood of stage 0 and a second variable corresponding to a likelihood of stage 1, stage 2, stage 3, and stage 4.

In step 416, the imputed value is provided in response to the request. The response in according to some embodiments comprises providing the imputed value to another process/computer. According to some embodiments, the response comprises storing the imputed value in the EHR system.

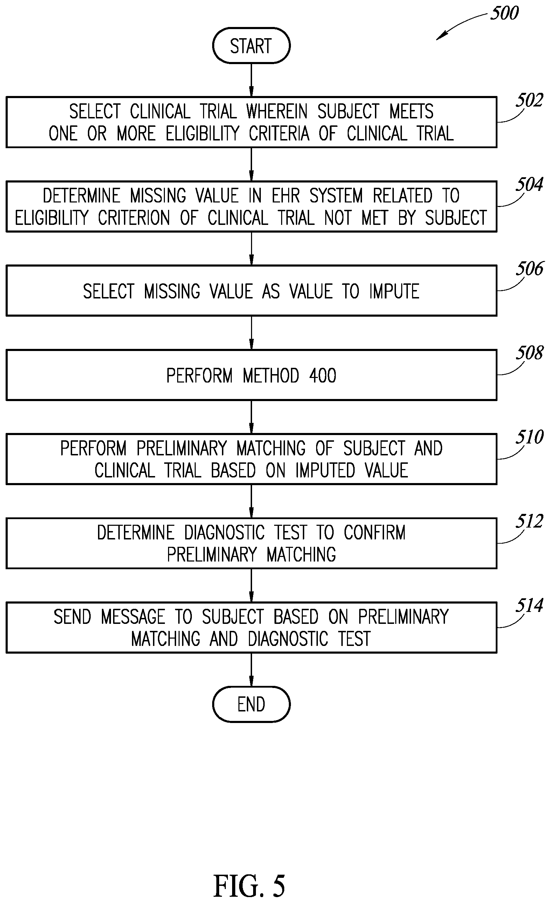

Figure 5 depicts a flowchart describing an embodiment of performing preliminary clinical trial matching using an imputed value.

Process 500 begins at step 502, wherein a clinical trial is selected, the clinical trial having one or more eligibility criteria met by a subject. According to some embodiments, the clinical trial is selected, and electronic health record system is searched for the subject meeting one or more eligibility criteria. According to some embodiments, a patient is selected from the electronic health record system and a clinical trial wherein the patient meets one or more eligibility criteria of the clinical trial is selected. According to some embodiments, both the subject and the clinical trial to be preliminarily matched are known. For example, the subject may provide the clinical trial they wish to participate in but currently fail to meet one or more eligibility criteria.

After step 502, at step 504 a missing value in the electronic health record system related to an eligibility criterion for the clinical trial not met by the subject is determined. According to some embodiments, the missing value directly corresponds to the eligibility criterion. When an eligibility criterion is a particular stage of cancer, for example, the patient meeting or not meeting the eligibility criterion can be preliminarily determined by imputing a missing cancer stage value. According to some embodiments, the missing value may not directly correspond with the eligibility criterion but may be used to support a finding of meeting or not meeting the eligibility criterion. For example, some eligibility criteria like liver function may not correspond directly to the missing value, but the missing value may be useful in determining whether the subject meets the eligibility criterion. Blood flow, epidemiological, environmental, and historical characteristics of the subject may be considered in determining whether the subject has normal liver function. But certain markers of liver function such as a score according to the Model for End-Stage Liver Disease scoring system may assist in assessing liver function, even though they may not be sufficient alone to assess whether the subject meets the eligibility criterion.

Following step 504, process 500 continues to step 506, wherein the missing value is selected as the value to impute.

In step 508, method 400 as described with respect to Figure 4 above, is performed using the selected missing value as the value to impute. Method 400 produces an imputed value as output.

After performing method 400 at step 508, in step 510 a preliminary matching of the subject and the clinical trial based on the imputed value is performed. As mentioned above, the imputed value may correspond directly to the eligibility criterion or be otherwise related to the eligibility criterion. In some embodiments wherein the imputed value directly corresponds to the eligibility criteria, the imputed value is compared to the eligibility criterion. For example, if the eligibility criterion is stage 3 or stage 4 cancer, the imputed value is checked to determine whether it corresponds to cancer stage 3 or stage 4.

As described above, the imputed value is according to some embodiments only correlated with or related to the eligibility criterion. In these cases, a relationship between the imputed value and the eligibility criterion may be used. For example, when the eligibility criterion is normal liver function, a bilirubin level may be the imputed value, where normal liver function is associated with a normal range of bilirubin levels. If the imputed value of bilirubin level is within the normal range of bilirubin levels, normal liver function is indicated. The relationship between the imputed value and the eligibility criterion may be inferred according to some embodiments using literature or a characteristic of the imputed value as compared to other measured and/or imputed values of the same feature in the EHR system. For example, the imputed value may be compared to other measured and/or imputed value of a same type in the EHR system. The imputed value may then be considered to indicate meeting or failing to meet the eligibility criteria based on a percentile of the imputed value as compared to the other measured and/or imputed values of a same type in the EHR system. A bilirubin value in a 99^{th} percentile as compared to other bilirubin values in the EHR system, for example, may indicate that the eligibility criterion of normal liver function is not met. Other metrics of comparison such as standard deviations of the imputed value from the mean of the other measured and/or imputed values of the same type in the EHR system may be used to indicate meeting or failing to meet an eligibility criterion. While the above description relates to imputing one value, any number of values may be imputed in a similar way.

In step 512, a diagnostic test to confirm the preliminary matching is determined. As discussed above, the imputed value is used to indicate whether an eligibility criterion is met. The diagnostic test according to some embodiments directly measures a characteristic of the subject predicted by the imputed value to confirm or refute the imputed value and whether the subject meets the eligibility criterion. According to some embodiments, the diagnostic test measures another characteristic of the subject related to the eligibility criterion.

In step 514, a message is sent to the subject based on the preliminary matching and the diagnostic test. According to some embodiments, a scheduling system of one or more healthcare providers is accessed to determine one or more available appointments for the diagnostic test to be performed on the subject. An appointment in the one or more appointments may then be preliminarily scheduled according to some embodiments, and the subject may be notified about the preliminarily scheduled appointment. According to some embodiments, the one or more appointments are included in the message, and the subject may select an appointment from the one or more appointments to schedule. According to some embodiments, in response to receiving a selection of the appointment from the subject, a second message is sent to the scheduling system of the healthcare provider to schedule the selected appointment.

For brevity and clarity, example embodiments discussed above include one subject being preliminarily matched with one clinical trial based on one imputed value. But any number of subjects may be preliminary matched with any number of clinical trials using any number of imputed missing values in a similar way. For example, a subject may be selected, and then one or more clinical trials may be iterated over, with each clinical trial for which the subject meets one or more eligibility criteria being selected. According to some embodiments, a clinical trial may be selected, and one or more patients may be iterated over, with the clinical trial being selected with respect to each subject meeting one or more eligibility criteria. According to some embodiments, preliminary matching of one subject and one clinical trial includes imputing a plurality of missing values.

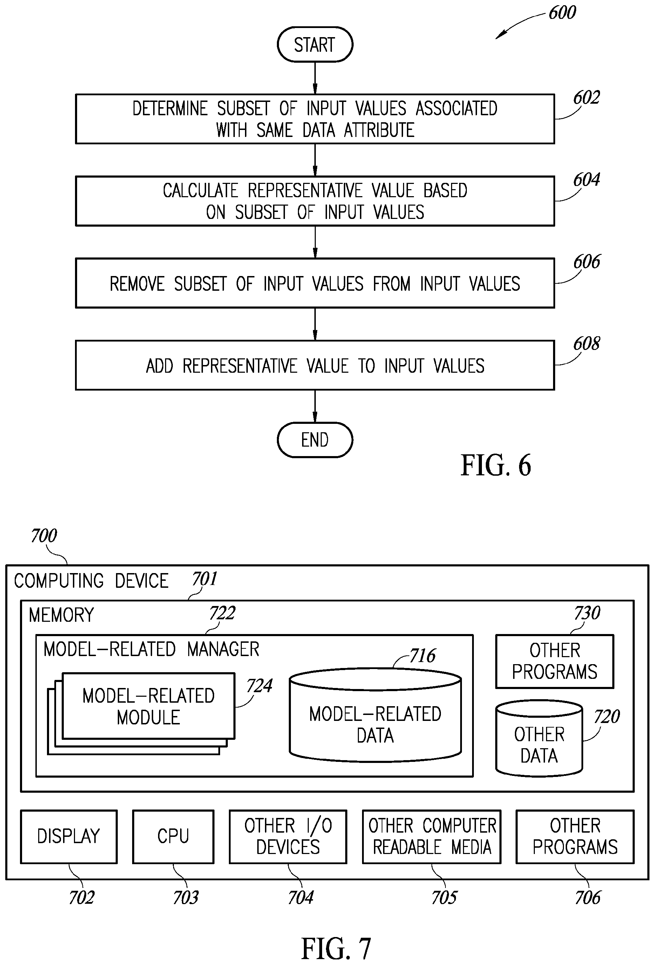

Figure 6 depicts a flowchart describing a process for aggregating a subset of input values into a representative value according to some embodiments. Process 600 begins at step 602, wherein each input value in a subset of input values is determined to be associated with a same data attribute. According to some embodiments, a data attribute is a feature, such as post_diag_immuno_check feature 326 shown in Figure 3C. For example, a feature in the subject feature vector may correspond to cancer stage. But a first input value may indicate stage 3 cancer, while a second input value may indicate stage 4 cancer. This may occur when the diagnostic window contains a plurality of observations relating to the same feature. The subject may have received several different diagnoses of cancer stage within the diagnostic window. Values corresponding to a plurality of observations of the same feature may be used to calculate a representative value.

In step 604, a representative value is calculated based on the subset of input values. According to some embodiments wherein the feature of the subset of input values is a cancer stage, a most severe cancer stage in the subset of input values is the representative value. According to some embodiments, a largest or smallest input value in the subset is the representative value. According to some embodiments, the representative value is a median, mode, mean, or weighted average of the input values in the subset. The representative value may also be the value in the subset corresponding to a health observation having an earliest or latest temporal instance.

Process 600 continues from step 604 to step 606, wherein the subset of input values is removed from the input values.

Continuing from step 606 to step 608, the calculated representative value is added to the input values. According to some embodiments, the representative value is inserted into the field of the subject feature vector in the location corresponding to the feature.

According to some embodiments, one or more general purpose or special purpose computing systems or devices may be used to implement the computing device 700. In addition, according to some embodiments, the computing device 700 may comprise one or more distinct computing systems or devices and may span distributed locations. Furthermore, each block shown in Figure 7 may represent one or more such blocks as appropriate to a specific embodiment or may be combined with other blocks. Also, the model-related manager 722 may be implemented in software, hardware, firmware, or in some combination to achieve the capabilities described herein.

As shown, the computing device 700 comprises a non-transitory computer memory ("memory") 701, a display 702 (including, but not limited to a light emitting diode (LED) panel, cathode ray tube (CRT) display, liquid crystal display (LCD), touch screen display, projector, etc.), one or more Central Processing Units ("CPU") or other processors 703, Input/Output ("I/O") devices 704 (e.g., keyboard, mouse, RF or infrared receiver, universal serial bus (USB) ports, High-Definition Multimedia Interface (HDMI) ports, other communication ports, and the like), other computer-readable media 705, and network connections 706. The model-related manager 722 is shown residing in memory 701. In other embodiments, some portion of the contents and some, or all, of the components of the model-related manager 722 may be stored on or transmitted over the other computer-readable media 705. The components of the computing device 700 and model-related manager 722 can execute on one or more CPUs 703 and implement applicable functions described herein. In some embodiments, the model-related manager 722 may operate as, be part of, or work in conjunction or cooperation with other software applications stored in memory 701 or on various other computing devices. In some embodiments, the model-related manager 722 also facilitates communication with peripheral devices via the I/O devices 704, or with another device or system via the network connections 706.

The one or more model-related modules 724 are configured to perform actions related, directly or indirectly, to AI or other computational model(s). In some embodiments, the model-related module(s) 724 stores, retrieves, or otherwise accesses at least some model-related data on some portion of the model-related data storage 716 or other data storage internal or external to the computing device 700.

Other code or programs 730 (e.g., further data processing modules, a program guide manager module, a Web server, and the like), and potentially other data repositories, such as data repository 720 for storing other data, may also reside in the memory 701, and can execute on one or more CPUs 703. Of note, one or more of the components in Figure 7 may or may not be present in any specific embodiment. For example, some embodiments may not provide other computer-readable media 705 or a display 702.

According to some embodiments, the computing device 700 and model-related manager 722 include API(s) that provides programmatic access to add, remove, or change one or more functions of the computing device 700. In some embodiments, components/modules of the computing device 700 and model-related manager 722 are implemented using standard programming techniques. For example, the model-related manager 722 may be implemented as an executable running on the CPU 703, along with one or more static or dynamic libraries. In other embodiments, the computing device 700 and model-related manager 722 may be implemented as instructions processed by a virtual machine that executes as one of the other programs 730. In general, a range of programming languages known in the art may be employed for implementing such example embodiments, including representative embodiments of various programming language paradigms, including but not limited to, object-oriented (*e.g*., Java, C++, C#, Visual Basic.NET, Smalltalk, and the like), functional (*e.g*., ML, Lisp, Scheme, and the like), procedural (*e.g.,* C, Pascal, Ada, Modula, and the like), scripting (*e.g.,* Perl, Ruby, Python, JavaScript, VBScript, and the like), or declarative (*e.g*., SQL, Prolog, and the like).

In a software or firmware embodiment, instructions stored in a memory configure, when executed, one or more processors of the computing device 700 to perform the functions of the model-related manager 722. In some embodiments, instructions cause the CPU 703 or some other processor, such as an I/O controller/processor, to perform at least some functions described herein.

The embodiments described above may also use well-known or other synchronous or asynchronous client-server computing techniques. However, the various components may be implemented using more monolithic programming techniques as well, for example, as an executable running on a single CPU computer system, or alternatively decomposed using a variety of structuring techniques known in the art, including but not limited to, multiprogramming, multithreading, client-server, or peer-to-peer, running on one or more computer systems each having one or more CPUs or other processors. Some embodiments may execute concurrently and asynchronously, and communicate using message passing techniques. Equivalent synchronous embodiments are also supported by a model-related manager 722 embodiment. Also, other functions could be implemented or performed by each component/module, and in different orders, and by different components/modules, yet still achieve the functions of the computing device 700 and model-related manager 722.

In addition, programming interfaces to the data stored as part of the computing device 700 and model-related manager 722, can be available by standard mechanisms such as through C, C++, C#, and Java APIs; libraries for accessing files, databases, or other data repositories; scripting languages such as XML; or Web servers, FTP servers, NFS file servers, or other types of servers providing access to stored data. The model-related data storage 716 and data repository 720 may be implemented as one or more database systems, file systems, or any other technique for storing such information, or any combination of the above, including embodiments using distributed computing techniques.

Different configurations and locations of programs and data are contemplated for use with techniques described herein. A variety of distributed computing techniques are appropriate for implementing the components of the illustrated embodiments in a distributed manner including but not limited to TCP/IP sockets, RPC, RMI, HTTP, and Web Services (XML-RPC, JAX-RPC, SOAP, and the like). Other variations are possible. Other functionality could also be provided by each component/module, or existing functionality could be distributed amongst the components/modules in different ways, yet still achieve the functions of the model-related manager 722.

Furthermore, according to some embodiments, some or all of the components of the computing device 700 and model-related manager 722 may be implemented or provided in other manners, such as at least partially in firmware or hardware, including, but not limited to one or more application-specific integrated circuits ("ASICs"), standard integrated circuits, controllers (*e.g*., by executing appropriate instructions, and including microcontrollers or embedded controllers), field-programmable gate arrays ("FPGAs"), complex programmable logic devices ("CPLDs"), and the like. Some or all of the system components or data structures may also be stored as contents (*e.g*., as executable or other machine-readable software instructions or structured data) on a computer-readable medium (*e.g*., as a hard disk; a memory; a computer network, cellular wireless network or other data transmission medium; or a portable media article to be read by an appropriate drive or via an appropriate connection, such as a DVD or flash memory device) so as to enable or configure the computer-readable medium or one or more associated computing systems or devices to execute or otherwise use, or provide the contents to perform, at least some of the described techniques.

Also disclosed herein are a number of examples, according to the following numbered clauses.

Clause 1. A method for imputing a value associated with a subject within an electronic health record (EHR) system, the method comprising: receiving a request to impute a value associated with the subject at a diagnostic temporal instance; retrieving a subset of data associated with the subject from the EHR system, the subset of data organized into specific fields as part of a schema and including a plurality of health observations, wherein each health observation is associated with a temporal instance; determining, for each health observation, a pre-diagnostic temporal window (206) and a post-diagnostic temporal window (208), the pre-diagnostic temporal window (206) comprising a specified first duration immediately preceding the diagnostic temporal instance (204), and the post-diagnostic temporal window (208) comprising a specified second duration immediately following the diagnostic temporal instance (204); selecting health observations having a temporal instance within the pre-diagnostic temporal window or the post-diagnostic temporal window (212, 214); retrieving, for each of the selected health observations (212, 214), a value corresponding to the selected health observation, as input values; providing the input values to a trained artificial intelligence engine (122), the trained artificial intelligence engine (122) configured to perform actions, comprising: processing the input values using the trained artificial intelligence engine (122) to generate the imputed value; and providing the imputed value (126) in response to the request.

Clause 2. The method of clause 1, wherein the value to impute is: a cancer stage; or a value relevant in making a risk determination; or a value related to an eligibility criterion of a clinical trial.

Clause 3. The method of any one of clauses 1-2, wherein determining the pre-diagnostic temporal window is based on: a characteristic of a condition associated with the value to be imputed; or a survival rate of a condition associated with the health observation; or a diagnostic window hyperparameter.

Clause 4. The method of any one of clauses 1-3, wherein the plurality of health observations includes one or more health observations selected based on a relevance to an extent of tumor, extent of spread to lymph nodes, or presence of metastasis (TNM) staging system.

Clause 5. The method of any one of clauses 1-4, further comprising determining a cancer stage based on the imputed value, wherein the cancer stage conforms with a TNM staging system, which assesses an extent of a tumor, a degree of spread to lymph nodes, and a presence of metastasis.

Clause 6. The method of any one of clauses 1-5, further comprising: selecting a health observation to include in the subset of data based on a variance of a value corresponding to a field in the health observation computed using a plurality of health observations in the EHR system having the field.

Clause 7. The method of any one of clauses 1-6, further comprising: receiving a request to impute a second value associated with the subject at a second diagnostic temporal instance; adding the imputed value to the input values; and providing the input values to the trained artificial intelligence engine, the trained artificial intelligence engine configured to perform actions, comprising: processing the input values using the trained artificial intelligence engine to generate the second imputed value; and providing the second imputed value.

Clause 8. The method of any one of clauses 1-7, further comprising: retrieving a second subset of data associated with the subject from the EHR system, the second subset of data organized into specific fields as part of a schema and including a plurality of second health observations, wherein each second health observation is not associated with a temporal instance; retrieving, for each second health observation in the second subset of data, a value corresponding to the selected health observation, as second input values; and adding the second input values to the input values.

Clause 9. The method of any one of clauses 1-8, further comprising: selecting a clinical trial wherein the subject meets one or more eligibility criteria of the clinical trial; determining a missing value in the data associated with the subject from the EHR system, wherein the missing value relates to an eligibility criterion for the clinical trial not met by the subject; selecting the missing value as the value to impute; automatically performing a preliminary matching of the subject with the clinical trial based on the imputed value; determining an appropriate diagnostic test to confirm the preliminary matching; and sending a message to the subject, wherein the message is based on the preliminary matching and the appropriate diagnostic test to confirm the preliminary matching.

Clause 10. The method of any one of clauses 1-9, further comprising: determining that each input value in a subset of the input values is associated with a same data attribute; calculating a representative value based on the subset of the input values; removing the subset of the input values from the input values; and adding the representative value to the input values.

Clause 11. The method of any one of clauses 1-10, further comprising: determining that each input value in a subset of the input values is associated with a same data attribute; calculating a temporal sub-window that comprises a portion of the pre-diagnostic temporal window and a portion of the post-diagnostic temporal window; determining a bucket of values, the bucket of values including each input value in the subset corresponding to a health observation having a temporal instance in the temporal sub-window; determining a count of the values in the bucket of values; removing the input values in the bucket of values from the subset; and adding the count to the subset.

Clause 12. The method of any one of clause 1 or clauses 3-11, further comprising: automatically performing a preliminary matching of the subject with a clinical trial, wherein the imputed value is a value related to an eligibility criterion of the clinical trial.

Clause 13. The method of any one of clauses 1-11, further comprising: automatically performing a preliminary matching of the subject with a clinical trial based on the imputed value.

Clause 14. A computing system for imputing a value associated with a subject within an electronic health record (EHR) system, the computing system comprising: one or more processors (703); and one or more non-transitory computer-readable media (705) collectively storing instructions that, when collectively executed by the one or more processors (703), cause the one or more processors (703) to perform actions, the actions comprising: receiving a request to impute a value (126) associated with the subject at a diagnostic temporal instance; retrieving a subset of data associated with the subject from the EHR system, the subset of data organized into specific fields as part of a schema and including a plurality of health observations, wherein each health observation is associated with a temporal instance; determining, for each health observation, a diagnostic temporal window (256) comprising a specified duration containing the diagnostic temporal instance (250); selecting health observations having a temporal instance within the diagnostic temporal window (264, 266); retrieving, for each of the selected health observations (264, 266), a value corresponding to the selected health observation, as input values; providing the input values to a trained artificial intelligence engine (122), the trained artificial intelligence engine (122) configured to perform actions, comprising: processing the input values using the trained artificial intelligence engine (122) to generate the imputed value; and providing the imputed value in response to the request.

Clause 15. A non-transitory processor-readable storage medium (705) that stores computer instructions that, when executed by a processor (703), cause the processor (703) to perform actions, the actions comprising: receiving a request to impute a value associated with a subject at a diagnostic temporal instance; retrieving a subset of data associated with the subject from an electronic health record (EHR) system, the subset of data organized into specific fields as part of a schema and including a plurality of health observations, wherein each health observation is associated with a temporal instance; determining, for each health observation, a diagnostic temporal window (256) comprising a specified duration containing the diagnostic temporal instance (250); selecting health observations having a temporal instance within the diagnostic temporal window (264, 266); retrieving, for each of the selected health observations (264, 266), a value corresponding to the selected health observation, as input values; providing the input values to a trained artificial intelligence engine (122), the trained artificial intelligence engine (122) configured to perform actions, comprising: processing the input values using the trained artificial intelligence engine (122) to generate the imputed value (126); and providing the imputed value (126) in response to the request.

The various embodiments described above can be combined to provide further embodiments. All of the U.S. patents, U.S. patent application publications, U.S. patent applications, foreign patents, foreign patent applications and non-patent publications referred to in this specification and/or listed in the Application Data Sheet are incorporated herein by reference, in their entirety. Aspects of the embodiments can be modified, if necessary to employ concepts of the various patents, applications and publications to provide yet further embodiments.

These and other changes can be made to the embodiments in light of the above-detailed description. In general, in the following claims, the terms used should not be construed to limit the claims to the specific embodiments disclosed in the specification and the claims but should be construed to include all possible embodiments along with the full scope of equivalents to which such claims are entitled. Accordingly, the claims are not limited by the disclosure.

## Claims

1. A method for imputing a value associated with a subject within an electronic health record (EHR) system, the method comprising:
receiving a request to impute a value associated with the subject at a diagnostic temporal instance;
retrieving a subset of data associated with the subject from the EHR system, the subset of data organized into specific fields as part of a schema and including a plurality of health observations, wherein each health observation is associated with a temporal instance;
determining, for each health observation, a pre-diagnostic temporal window (206) and a post-diagnostic temporal window (208), the pre-diagnostic temporal window (206) comprising a specified first duration immediately preceding the diagnostic temporal instance (204), and the post-diagnostic temporal window (208) comprising a specified second duration immediately following the diagnostic temporal instance (204);
selecting health observations having a temporal instance within the pre-diagnostic temporal window or the post-diagnostic temporal window (212, 214);
retrieving, for each of the selected health observations (212, 214), a value corresponding to the selected health observation, as input values;
providing the input values to a trained artificial intelligence engine (122), the trained artificial intelligence engine (122) configured to perform actions, comprising:
processing the input values using the trained artificial intelligence engine (122) to generate the imputed value; and
providing the imputed value (126) in response to the request.

2. The method of claim 1, wherein the value to impute is:
a cancer stage; or
a value relevant in making a risk determination; or
a value related to an eligibility criterion of a clinical trial.

3. The method of any one of claims 1-2, wherein determining the pre-diagnostic temporal window is based on:
a characteristic of a condition associated with the value to be imputed; or
a survival rate of a condition associated with the health observation; or
a diagnostic window hyperparameter.

4. The method of any one of claims 1-3, wherein the plurality of health observations includes one or more health observations selected based on a relevance to an extent of tumor, extent of spread to lymph nodes, or presence of metastasis (TNM) staging system.

5. The method of any one of claims 1-4, further comprising determining a cancer stage based on the imputed value, wherein the cancer stage conforms with a TNM staging system, which assesses an extent of a tumor, a degree of spread to lymph nodes, and a presence of metastasis.

6. The method of any one of claims 1-5, further comprising:
selecting a health observation to include in the subset of data based on a variance of a value corresponding to a field in the health observation computed using a plurality of health observations in the EHR system having the field.

7. The method of any one of claims 1-6, further comprising:
receiving a request to impute a second value associated with the subject at a second diagnostic temporal instance;
adding the imputed value to the input values; and
providing the input values to the trained artificial intelligence engine, the trained artificial intelligence engine configured to perform actions, comprising:
processing the input values using the trained artificial intelligence engine to generate the second imputed value; and
providing the second imputed value.

8. The method of any one of claims 1-7, further comprising:
retrieving a second subset of data associated with the subject from the EHR system, the second subset of data organized into specific fields as part of a schema and including a plurality of second health observations, wherein each second health observation is not associated with a temporal instance;
retrieving, for each second health observation in the second subset of data, a value corresponding to the selected health observation, as second input values; and
adding the second input values to the input values.

9. The method of any one of claims 1-8, further comprising:
selecting a clinical trial wherein the subject meets one or more eligibility criteria of the clinical trial;
determining a missing value in the data associated with the subject from the EHR system, wherein the missing value relates to an eligibility criterion for the clinical trial not met by the subject;
selecting the missing value as the value to impute;
automatically performing a preliminary matching of the subject with the clinical trial based on the imputed value;
determining an appropriate diagnostic test to confirm the preliminary matching; and
sending a message to the subject, wherein the message is based on the preliminary matching and the appropriate diagnostic test to confirm the preliminary matching.

10. The method of any one of claims 1-9, further comprising:
determining that each input value in a subset of the input values is associated with a same data attribute;
calculating a representative value based on the subset of the input values;
removing the subset of the input values from the input values; and
adding the representative value to the input values.

11. The method of any one of claims 1-10, further comprising:
determining that each input value in a subset of the input values is associated with a same data attribute;
calculating a temporal sub-window that comprises a portion of the pre-diagnostic temporal window and a portion of the post-diagnostic temporal window;
determining a bucket of values, the bucket of values including each input value in the subset corresponding to a health observation having a temporal instance in the temporal sub-window;
determining a count of the values in the bucket of values;
removing the input values in the bucket of values from the subset; and
adding the count to the subset.

12. The method of any one of claim 1 or claims 3-11, further comprising:
automatically performing a preliminary matching of the subject with a clinical trial, wherein the imputed value is a value related to an eligibility criterion of the clinical trial.

13. The method of any one of claims 1-11, further comprising:
automatically performing a preliminary matching of the subject with a clinical trial based on the imputed value.

14. A computing system for imputing a value associated with a subject within an electronic health record (EHR) system, the computing system comprising:
one or more processors (703); and
one or more non-transitory computer-readable media (705) collectively storing instructions that, when collectively executed by the one or more processors (703), cause the one or more processors (703) to perform actions, the actions comprising:
receiving a request to impute a value (126) associated with the subject at a diagnostic temporal instance;
retrieving a subset of data associated with the subject from the EHR system, the subset of data organized into specific fields as part of a schema and including a plurality of health observations, wherein each health observation is associated with a temporal instance;
determining, for each health observation, a diagnostic temporal window (256) comprising a specified duration containing the diagnostic temporal instance (250);
selecting health observations having a temporal instance within the diagnostic temporal window (264, 266);
retrieving, for each of the selected health observations (264, 266), a value corresponding to the selected health observation, as input values;
providing the input values to a trained artificial intelligence engine (122), the trained artificial intelligence engine (122) configured to perform actions, comprising:
processing the input values using the trained artificial intelligence engine (122) to generate the imputed value; and
providing the imputed value in response to the request.

15. A non-transitory processor-readable storage medium (705) that stores computer instructions that, when executed by a processor (703), cause the processor (703) to perform actions, the actions comprising:
receiving a request to impute a value associated with a subject at a diagnostic temporal instance;
retrieving a subset of data associated with the subject from an electronic health record (EHR) system, the subset of data organized into specific fields as part of a schema and including a plurality of health observations, wherein each health observation is associated with a temporal instance;
determining, for each health observation, a diagnostic temporal window (256) comprising a specified duration containing the diagnostic temporal instance (250);
selecting health observations having a temporal instance within the diagnostic temporal window (264, 266);
retrieving, for each of the selected health observations (264, 266), a value corresponding to the selected health observation, as input values;
providing the input values to a trained artificial intelligence engine (122), the trained artificial intelligence engine (122) configured to perform actions, comprising:
processing the input values using the trained artificial intelligence engine (122) to generate the imputed value (126); and
providing the imputed value (126) in response to the request.
